# EUROPÄISCHE PATENTANMELDUNG

(11) **EP 3 910 605 A2**
(43) Veröffentlichungstag der Anmeldung: **17.11.2021**
(21) Anmeldenummer: 21173467.8
(22) Anmeldetag: 12.05.2021
(51) Int. Cl.: G07C 9/00, G06K 19/06, G07C 9/22, G06Q 50/26, G16H 10/40, G16H 50/80

(54) **VERFAHREN ZUR ERZEUGUNG EINES SICHERHEITSDOKUMENTS UND VERWENDUNG DES SICHERHEITSDOKUMENTS UND SICHERHEITSSYSTEM**

(30) Priorität: 15.05.2020 DE 102020113311
(71) Anmelder: Bundesdruckerei GmbH, 10969 Berlin (DE)
(72) Erfinder: BASTIAN, Paul, 10243 Berlin (DE); BÖSCH, Christoph, 14558 Nuthetal (DE); FISCHER, Jörg, 10317 Berlin (DE); GRAUPNER, Hendrik, 14050 Berlin (DE); KRAUS, Micha, 10827 Berlin (DE); MORGNER, Frank, 15537 Grünheide (DE); MUSICK, Robert, 12167 Berlin (DE); TIETKE, Markus, 12439 Berlin (DE); WEDEKIND, Torben, 15732 Eichwalde (DE)
(74) Vertreter: Richardt Patentanwälte PartG mbB

(57) **Zusammenfassung**

Die Erfindung betrifft ein Verfahren zur Erzeugung eines Sicherheitsdokuments (110) für eine Person (100), die Inhaber eines Ausweisdokuments (102) ist, mit folgenden Schritten:
- Auslesen von Identitätsdaten der Person aus dem Ausweisdokument,
- Eingabe einer der Person zugeordneten Information (118),
- Erzeugung eines Datensatzes aus den Identitätsdaten und der der Person zugeordneten Information,
- Ausgabe des Datensatzes als Sicherheitsdokument (110), das den Datensatz als maschinenlesbaren Code (120; 124) beinhaltet.

## Beschreibung

Die Erfindung betrifft das Gebiet der Ausweisdokumente, insbesondere der maschinenlesbaren Ausweisdokumente.

Ausweisdokumente, wie zum Beispiel Personalausweise und Reisepässe, beinhalten optisch lesbare Personalisierungsinformationen, die den Inhaber des Ausweisdokuments eindeutig identifizieren. Solche Informationen beinhaltet auch die sogenannte maschinenlesbare Zone (Machine Readable Zone - MRZ), wie sie für maschinenlesbare Ausweise (machine readable travel documents - MRTD) von der internationalen Luftfahrtbehörde standardisiert sind. Auch der elektronische Personalausweis der Bundesrepublik Deutschland beinhaltet eine solche MRZ.

Solche an sich bekannte Ausweisdokumente werden für hoheitliche Zwecke, wie zum Beispiel für die Grenzkontrolle, zur Identitätsprüfung eingesetzt. Als weitere Ausweisdokumente eignen sich Mitarbeiterausweise, Gesundheitskarten, Bankkarten oder andere Eintrittskarten.

Der Erfindung liegt demgegenüber die Aufgabe zugrunde, eine weitere Verwendungsmöglichkeit für solche Ausweisdokumente zu schaffen.

Die der Erfindung zugrunde liegende Aufgabe wird mit den Merkmalen der unabhängigen Ansprüche gelöst. Ausführungsformen der Erfindung sind in den abhängigen Patentansprüchen angegeben.

Ausführungsformen der Erfindung sind besonders vorteilhaft, da die Zuordnung einer Information zu einer Person, die Inhaber des Ausweisdokuments ist, auf sicherer und nachprüfbarer Art und Weise erfolgt, die dem Sicherheitsniveau des Ausweisdokuments entspricht, obwohl die Information nicht Teil des Ausweisdokuments ist.

Dies wird dadurch ermöglicht, dass die Information in ein Sicherheitsdokument eingebracht wird, welches dem Ausweisdokument eindeutig und unveränderlich zugeordnet ist. Diese Zuordnung erfolgt, indem zur Erzeugung des Sicherheitsdokuments zunächst die Identitätsdaten der Person aus dem Ausweisdokument ausgelesen werden, beispielsweise durch Erfassung der MRZ. Aus den Identitätsdaten des Ausweisdokuments und der der Person zugeordneten Information wird dann ein Datensatz erzeugt, beispielsweise indem die Identitätsdaten und die Information miteinander kombiniert werden. Dieser Datensatz wird dann als Sicherheitsdokument ausgegeben, das den Datensatz als maschinenlesbaren Code beinhaltet.

Nach einer Ausführungsform der Erfindung ist die Information und vorzugsweise auch die Identitätsdaten zusätzlich im Klartext auf dem Sicherheitsdokument angegeben.

Zur Prüfung der Information auf Authentizität kann so vorgegangen werden, dass der maschinenlesbare Code zum Beispiel mit dem Endgerät, beispielsweise einem Smartphone, oder einer Zugangskontrollvorrichtung gelesen wird. Ferner werden auch die Identitätsdaten der Person aus dem Ausweisdokument gelesen, z.B. durch eine Sichtprüfung oder maschinell. Wenn die Identitätsdaten aus dem Ausweisdokument mit den Identitätsdaten aus dem Datensatz des Sicherheitsdokuments übereinstimmen, so gilt auch die in dem Datensatz beinhaltete Information als authentisch.

Nach Ausführungsformen der Erfindung kann die Information hinsichtlich eines Kriteriums überprüft werden, um beispielsweise dem Träger des Ausweisdokuments Zugang zu einem zugangsbeschränkten Bereich zu gewähren.

Nach Ausführungsformen der Erfindung kann die Information einen medizinisch Zustand der Person, einen Identifikator eines der Person zugeordneten Gegenstandes (der sich z.B. erlaubterweise im Besitz oder Eigentum der Person befinden darf), eine Angabe einer Befähigung oder Berechtigung der Person (z.B. ein Ticket für den Besuch einer Veranstaltung oder die Nutzung eines Verkehrsmittels) und/oder eine zeitliche Begrenzung und/oder mehrere solcher Angaben beinhalten.

In einem weiteren Aspekt betrifft die Erfindung eine Verwendung eines Sicherheitsdokuments zum Schutz vor der Ausbreitung einer Infektionskrankheit, insbesondere im Falle einer Epidemie oder Pandemie. Bei der Information kann es sich in diesem Fall um die Angabe handeln, dass der Inhaber des Ausweisdokuments und damit auch des Sicherheitsdokuments, zum Zeitpunkt einer Untersuchung nicht an einer Infektionskrankheit erkrankt war. Der Inhaber des Ausweisdokuments ist daher wahrscheinlich während eines gewissen Zeitraums nicht infektiös, selbst wenn er sich nach der Untersuchung infizieren sollte. Hintergrund ist, dass es nach einer Infektion eine gewisse Zeit dauert, bis der Infizierte andere infizieren kann. Vorzugsweise wird diese Information, d.h. dass zum Zeitpunkt der Untersuchung keine Erkrankung an der Infektionskrankheit vorlag, ergänzt, um eine Angabe dieses wahrscheinlichen Zeitraums, wobei dieser Zeitraum als die wahrscheinliche Gültigkeitsdauer dafür betrachtet werden kann, dass der Inhaber nicht ansteckend ist. Mit anderen Worten kann es sich bei der Information um die Angabe handeln, dass der Inhaber bis zu einem bestimmten Zeitpunkt nicht ansteckend ist.

Bei der Infektionskrankheit kann es sich um eine Krankheit handeln, die über die Atmung oder Berührung übertragbar ist, insbesondere eine Krankheit, die hochinfektiös ist, wie z.B. 2019-nCoV, COVID-19, COVID-19, d.h. der Coronavirus, oder "Schweres akutes Atemwegssyndrom" oder "Schweres akutes respiratorisches Syndrom"(SARS) oder eine existente oder zukünftige Mutation davon.

Möchte der Inhaber des Ausweisdokuments beispielsweise ein Firmengebäude oder eine Bildungseinrichtung betreten, oder an einer Großveranstaltung teilnehmen oder eine Reise unternehmen, so lässt er sich zuvor auf eine Infektion mit der Infektionskrankheit, beispielsweise durch den Coronavirus, testen. Liegt eine solche Infektion nicht vor, so wird beispielsweise von dem Labor, welches die Prüfung auf die Infektion durchgeführt hat, ein erfindungsgemäßes Sicherheitsdokument für den Inhaber des Ausweisdokuments ausgestellt.

Mithilfe dieses Sicherheitsdokuments in Kombination mit seinem Ausweisdokument kann dann dem Inhaber des Ausweisdokuments Zutritt zu einem zutrittsbeschränkten Bereich, beispielsweise einer Großveranstaltung oder eines öffentlichen Verkehrsmittels, wie zum Beispiel einem Passagierflugzeug, gewährt werden. Entsprechendes gilt für die Grenzkontrolle bei der Ausreise oder Einreise, bei dem der Inhaber das Ergebnis eines Tests nachweisen kann. Wenn eine solche Kontrolle systematisch möglichst für sämtliche Teilnehmer z.B. der Großveranstaltung durchgeführt wird, so ist damit sichergestellt, dass sich die Infektionskrankheit in dem entsprechenden Teilnehmerkreis während der Großveranstaltung nicht weiterverbreiten kann, so dass ein Schutz vor der Ausbreitung der Infektionskrankheit hierdurch gegeben ist.

Ausführungsformen der Erfindung sind besonders vorteilhaft, da weder für die Ausstellung des Sicherheitsdokuments noch für dessen Prüfung zwingend eine Verbindung mit einem Kommunikationsnetzwerk, wie zum Beispiel dem Internet, erforderlich ist. Sowohl Ausstellung als auch Prüfung des Sicherheitsdokuments können komplett offline erfolgen.

Ausführungsformen der Erfindung sind ferner besonders vorteilhaft, da der Inhaber des Ausweisdokuments und des zugeordneten Sicherheitsdokuments die Hoheit über seine eigenen Daten behält, die nirgends zentral gespeichert werden müssen. Dies ist besonders für sensible Daten, die den medizinischen Zustand der Person betreffen und die besonders schutzwürdig sind, von besonderem Vorteil.

Ausführungsformen der Erfindung sind ferner besonders vorteilhaft, da das Sicherheitsdokument in Form eines Ausdrucks vorliegen kann. Dies kommt Nutzergruppen entgegen, die entweder keinen Zugriff auf ein elektronisches Endgerät, wie zum Beispiel ein Smartphone, haben, oder denen die Bedienung eines solchen elektronischen Endgeräts schwerfällt oder nicht möglich ist. Für solche Nutzergruppen haben Ausführungsformen der Erfindung Handhabungsvorteile, da sie zur Prüfung lediglich ihr Ausweisdokument zusammen mit dem Sicherheitsdokument vorlegen müssen, so wie sie es für eine herkömmliche Sichtprüfung ihres Ausweisdokuments zum Beispiel bei einer Grenzkontrolle gewohnt sind.

Nach Ausführungsformen der Erfindung liegt das Sicherheitsdokument elektronisch vor, beispielsweise als PDF-Dokument oder in einem anderen elektronischen Dokumentenformat. Ein solches elektronisches Sicherheitsdokument kann von dem Aussteller des Sicherheitsdokuments an ein mobiles Endgerät des Inhabers des Ausweisdokuments, wie zum Beispiel an dessen Smartphone, auf elektronischem Wege, wie zum Beispiel per E-Mail, Bluetooth oder durch Zurverfügungstellung eines Downloadlinks, übertragen werden. Dies hat den besonderen Vorteil, dass der Inhaber des Ausweisdokuments kein weiteres physisches Dokument bei sich führen muss, sondern für die Kontrolle des Sicherheitsdokuments lediglich seinen Ausweis und sein Smartphone mit dem geöffneten elektronischen Sicherheitsdokument präsentieren muss.

Nach einer weiteren Ausführungsform liegt das Ausweisdokument digital vor mit lesbaren Personalisierungsinformationen, die den Inhaber des Ausweisdokuments eindeutig identifizieren. Dies kann in einer sogenannten Wallet auf dem Smartphone des Ausweisdokumenteninhabers abgelegt sein.

Im Weiteren werden Ausführungsformen der Erfindung mit Bezugnahme auf die Zeichnungen näher erläutert. Es zeigen:
- Figur 1: ein Sicherheitssystem,
- Figur 2: ein Flussdiagramm,
- Figur 3: eine Ausführungsform der Erfindung, bei der es sich bei dem Aussteller des Sicherheitsdokuments um ein Labor handelt,
- Figur 4: eine Anwendung des Sicherheitsdokuments für eine Zugangskontrolle.

In der nachfolgenden Figurenbeschreibung werden einander entsprechende Elemente der verschiedenen Ausführungsformen mit gleichen Bezugszeichen gekennzeichnet.

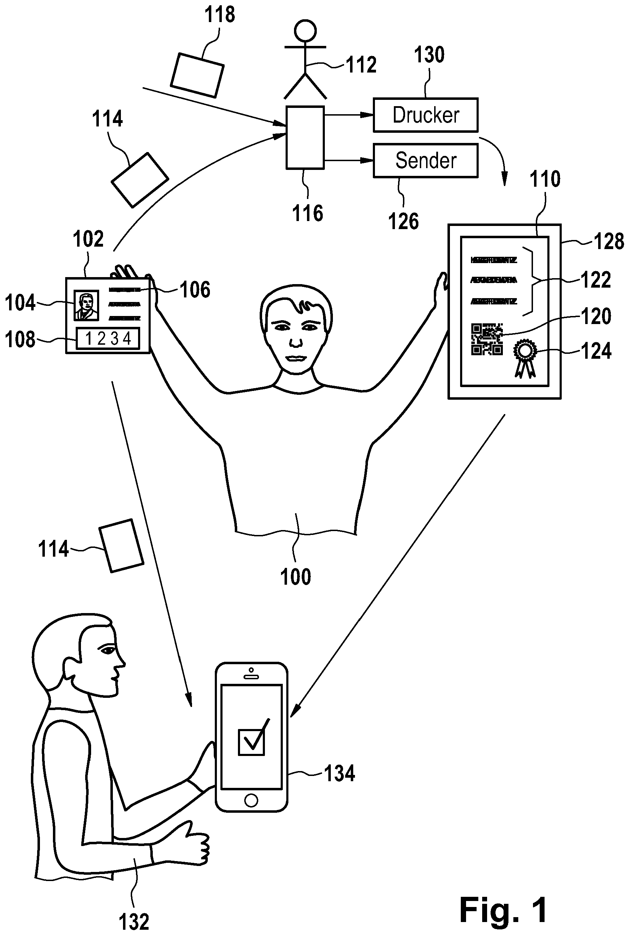

Die Figur 1 zeigt eine Person 100, die Inhaber eines Ausweisdokuments 102 ist. Bei dem Ausweisdokument 102 handelt es sich beispielsweise um ein maschinenlesbares Reisedokument, beispielsweise den Personalausweis der Bundesrepublik Deutschland. Das Ausweisdokument 102 beinhaltet ein Lichtbild 104 der Person 100 sowie Personalisierungsinformationen 106, die die Identität der Person 100 angeben, insbesondere den Namen, Vornamen, Geburtstag, etc. Diese Personalisierungsinformationen 106 sind im Klartext auf dem Ausweisdokument 102 vorhanden und sind daher durch eine Sichtprüfung zugänglich. Ferner hat das Ausweisdokument 102 eine Machine Readable Zone MRZ 108. Die MRZ 108 beinhaltet laut internationalem Standard die folgenden Daten: Dokumententyp, Seriennummer, Geburtstag, gültig bis, Name, Vornamen.

Zur Erzeugung eines Sicherheitsdokuments 110, welches der Person 100 und dem Ausweisdokument 102 eindeutig zugeordnet ist, wird nun wie folgt vorgegangen:
Ein Aussteller 112 des Sicherheitsdokuments 110 liest Personalisierungsinformationen von dem Ausweisdokument 102, die eine eindeutige Feststellung der Identitätsdaten der Person ermöglichen, wie zum Beispiel Geburtstag, Name, Vornamen. Dies kann durch Lesen der im Klartext vorhandenen Personalisierungsinformation 106 oder vorzugsweise durch Lesen der MRZ 108, das heißt durch einem sogenannten MRZ-Scan, erfolgen. Falls das Ausweisdokument 102 über einen integrierten Chip verfügt, kann gemäß einer weiteren Ausführungsform die Personalisierungsinformation auch aus dem Chip ausgelesen werden.

Diese aus dem Ausweisdokument 102 optisch oder elektronisch ausgelesenen Identitätsdaten 114, also zum Beispiel Geburtstag, Name, Vornamen, werden in ein elektronisches Gerät 116, wie zum Beispiel einen Computer, des Ausstellers 112 eingegeben oder übertragen. Ferner wird in das elektronische Gerät 116 auch eine der Person 100 von dem Aussteller 112 zugeordnete Information 118 eingegeben.

Bei dieser Information kann es sich beispielsweise um ein Testergebnis einer medizinischen Untersuchung handeln, wie zum Beispiel das Ergebnis einer Polymerase-Chain-Reaction (PCR)-Analyse, welche als Information 118 liefert, ob die Person 100 mit einer Infektionskrankheit, wie zum Beispiel dem Coronavirus, infiziert ist. Wenn keine Infektion der Person 100 festgestellt wird, dann folgt daraus, dass die Person aktuell und wahrscheinlich während eines Zeitraums von zwei bis drei Tagen nicht ansteckend ist, selbst wenn sich die Person 100 nach der Untersuchung infiziert. Als Information 118 kann also durch den Aussteller 112 eingegeben werden, dass die Person 100 nicht ansteckend ist und die entsprechende Gültigkeitsdauer, für die dieser Zustand mit hinreichender Sicherheit bestehen bleibt.

Es kann sich auf um eine andere Information handeln, wie z.B. eine Berechtigung der Person 100.

Durch das elektronische Gerät 116 wird ein Datensatz aus den Identitätsdaten 114 und der Information 118 erzeugt, indem die Identitätsdaten 114 und die Information 118 miteinander kombiniert werden, zum Beispiel aneinandergehängt (konkateniert) werden, oder auf eine andere Art und Weise miteinander verknüpft werden, insbesondere kryptografisch miteinander verknüpft werden.

Der so erzeugte Datensatz wird dann als das Sicherheitsdokument 110 ausgegeben, welches diesen Datensatz als maschinenlesbaren Code 120, wie zum Beispiel als QR-Code, Barcode, HEX-code oder Binärcode beinhaltet. Neben dem maschinenlesbaren Code 120 kann das Sicherheitsdokument 110 die Identitätsdaten 114 sowie die Information 118 auch als Klartext 122 beinhalten.

Vorzugsweise werden der von dem elektronischen Gerät 116 erzeugte Datensatz und/oder das Sicherheitsdokument 110 durch den Aussteller 112 elektronisch signiert und/oder mit einem elektronischen Siegel versehen, wobei der Aussteller 112 hierzu ein ihm oder seiner Organisation zugeordnetes Zertifikat einer Public Key Infrastructure (PKI) verwendet. In diesem Fall hat das Sicherheitsdokument 110 die genannte Signatur bzw. das Siegel 124, welches in dem maschinenlesbaren Code 120 beinhaltet sein kann. Die Signatur bzw. das Siegel 124 kann auch als ein weiterer maschinenlesbaren Code auf dem Dokument abgebildet sein zusätzlich zu dem maschinenlesbaren Code 120.

Das elektronische Gerät 116 kann über einen Sender 126 verfügen, wie zum Beispiel ein E-Mail-Programm oder eine Bluetooth-Schnittstelle, um das Sicherheitsdokument 110 als elektronisches Dokument an ein Endgerät 128 zu senden. Bei dem Endgerät 128 kann es sich zum Beispiel um ein mobiles batteriebetriebenes Telekommunikationsgerät, wie zum Beispiel ein sogenanntes Smartphone, der Person 100 handeln.

Alternativ oder zusätzlich kann das Sicherheitsdokument 110 von einem Drucker 130, der mit dem elektronischen Gerät 116 gekoppelt ist, ausgedruckt und an die Person 100 übergeben oder postalisch zugeschickt werden.

Zur Prüfung des Sicherheitsdokuments 110 kann zu einem späteren Zeitpunkt wie folgt vorgegangen werden:
Die Person 100 präsentiert ihr Ausweisdokument 102 und ihr Sicherheitsdokument 110 gegenüber einen Prüfer 132, der über ein elektronisches Gerät 134, wie zum Beispiel ein Smartphone, verfügt. Der Prüfer 132 erfasst mittels seinem elektronischen Gerät 134 die Identitätsdaten 114 von dem Ausweisdokument 102 in analoger Art und Weise, so wie es auch mithilfe des elektronischen Geräts 116 von dem Aussteller 112 durchgeführt worden ist.

Der Prüfer 132 erfasst ferner den maschinenlesbaren Code 120 von dem Sicherheitsdokument 110, beispielsweise mit der Kamera seines Smartphones und einer QR-Code Lese-App. Hierbei wird auch die Signatur/Siegel 124 durch das elektronische Gerät 134 erfasst und auf Validität geprüft. Das elektronische Gerät 134 überprüft dann die von dem Ausweisdokument 102 erfassten Identitätsdaten 114 auf Übereinstimmung mit den Identitätsdaten, die der maschinenlesbare Code 120 beinhaltet. Liegt eine solche Übereinstimmung vor, so wird die Authentizität der Information 118 signalisiert. Wenn das Sicherheitsdokument 110 von der Person 100 mit Hilfe des Endgeräts 128 präsentiert wird, so authentifiziert sich die Person 100 vorzugsweise zunächst gegenüber dem Endgerät 128 bevor das Sicherheitsdokument 110 angezeigt werden kann. Nach einer Ausführungsform zeigt das elektronische Gerät auch die Information 118 an, welche aus dem maschinenlesbare Code 120 vom dem elektronischen Gerät ausgelesen wurde; die Information 118 kann im Klartext auch auf dem Sicherheitsdokument 110 stehen.

Nach einer alternativen Ausführungsform werden die Identitätsdaten 114 nicht von dem elektronischen Gerät 134 auf Übereinstimmung mit den Identitätsdaten des maschinenlesbaren Codes 120 geprüft, sondern diese Prüfung wird von dem Prüfer 132 durchgeführt, und zwar durch eine Sichtprüfung des Ausweisdokuments 102 im Vergleich zu den in dem maschinenlesbaren Code 120 beinhalteten Identitätsdaten, die von dem elektronischen Gerät 134 angezeigt werden.

Nach Ausführungsformen der Erfindung wird zur Prüfung der Validität der Signatur/Siegel 124 des Sicherheitsdokuments 110 durch das elektronische Gerät 134 eine Zertifikatskettenprüfung durchgeführt.

In einer Ausführungsform der Erfindung handelt es sich bei dem elektronischen Gerät 134 um eine vollautomatische Zugangskontrollvorrichtung. Stimmen die Identitätsdaten 114, die von dem Ausweisdokument 102 erfasst wurden, mit den Identitätsdaten aus dem maschinenlesbaren Code 120 überein und ist auch die Signatur/Siegel 124 valide, so erzeugt das elektronische Gerät 134 ein Steuerungssignal für eine Zugangskontrollvorrichtung, die den Zugang der Person 100 zu einem zugangsbeschränkten Bereich freigibt.

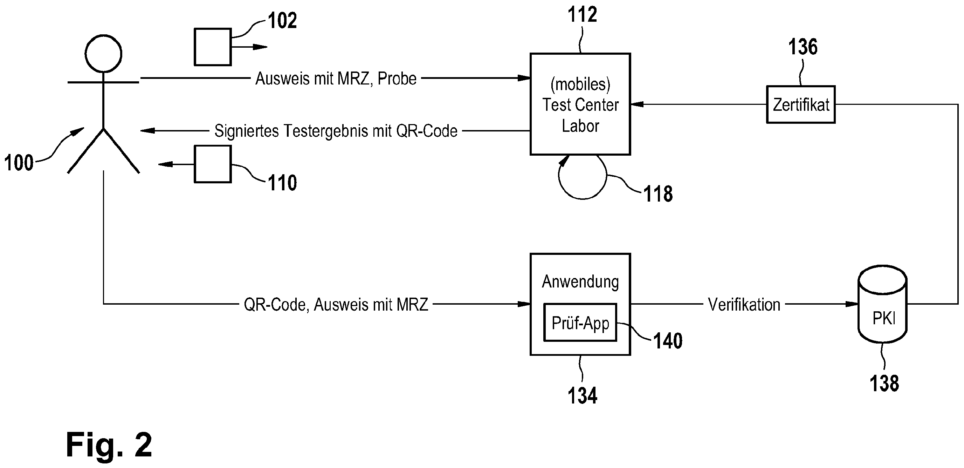

Die Figur 2 zeigt eine Ausführungsform der Erfindung, bei der sich die Person 100 zu dem Aussteller 112 begibt, bei dem es sich hier um ein Labor zur Feststellung eines medizinischen Zustands der Person handelt, insbesondere um ein mobiles Testcenterlabor, zur Prüfung, ob sich die Person 100 mit dem Coronavirus infiziert hat.

Hierzu wird der Person 100 zum Beispiel durch einen Abstrich eine Probe entnommen, die in dem Labor untersucht wird. Diese Information kann als Ergebnis die Information 118 haben, die dann nämlich besagt, dass sich die Person 100 nicht mit dem Coronavirus infiziert hat, so dass die Person selbst zum Zeitpunkt der Untersuchung und während einer gewissen Zeitdauer danach andere nicht infizieren kann. Dieser Information, dass sich also die Person 100 nicht infiziert hat, wird vorzugsweise noch die Gültigkeitsinformation bezüglich dieser Zeitdauer hinzugefügt und auf der Basis dieser gesamten Information 118 wird dann durch Kombination mit den Identitätsdaten 114 das Sicherheitsdokument 110 generiert, wie oben mit Bezugnahme auf Figur 1 dargestellt. Die Kombination kann durch einfaches aneinanderhängen (Konkatenieren) der Identitätsdaten 114 und der Information 118 erfolgen. Die Kombination kann aber auch nach einem komplexeren vordefinierten und reversiblen Verfahren durchgeführt werden.

Das elektronische Gerät 134 ist für die Prüfung dann dazu konfiguriert, dieses Verfahren durchzuführen, um die Kombination wieder rückgängig zu machen, d.h. um damit die Information 118 und die Identitätsdaten 114 aus der Kombination, die in dem maschinenlesbaren Code beinhaltet ist, zu ermitteln.

Für die Erzeugung der Signatur/Siegel 124 verwendet der Aussteller 112 ein Zertifikat 136 einer PKI 138. Das Zertifikat 136 beinhaltet einen öffentlichen Schlüssel des Ausstellers 112 eines kryptografischen Schlüsselpaares bestehend aus diesem öffentlichen Schlüssel und einem zugeordneten privaten Schlüssel mithilfe dessen die Signatur/Siegel 124 von dem Aussteller 112 erzeugt wird. Das Zertifikat 136 kann eine Angabe einer hoheitlichen Autorisierung des Ausstellers 112 für die Ausstellung des Sicherheitsdokuments 110 beinhalten. Vorzugsweise beinhaltet die Signatur/Siegel 124 auch das Zertifikat 136.

Nach Ausführungsformen der Erfindung wird durch den Prüfer 132 durch Einsichtnahme in das Zertifikat 136 auch geprüft, ob der Aussteller 112 die zur Ausstellung des Sicherheitsdokuments 110 erforderliche hoheitliche Ermächtigung hat und/oder diese Prüfung wird durch das elektronische Gerät 134 vollautomatisch durchgeführt.

Möchte die Person 100 nun zum Beispiel Zugang zu einem zugangsbeschränkten Bereich haben, den nur Personen betreten dürfen, die nicht mit einer Infektionskrankheit infiziert sind, so kann wie folgt vorgegangen werden:
Die Person 100 begibt sich zu dem elektronischen Gerät 134, welches hier als Zugangskontrollvorrichtung ausgebildet sein kann. Auf dem elektronischen Gerät 134 ist eine Prüf-App 140 installiert. Durch Ausführung der Prüf-App 140 werden die Identitätsdaten 114 aus dem Ausweisdokument 102 der Person 100 gelesen, und zwar je nach Ausführungsform optisch, beispielsweise durch einen MRZ-Scan, oder durch einen Lesezugriff auf einen Speicherchip, der in dem Ausweisdokument 102 integriert ist und der die Identitätsdaten 114 beinhaltet. Andererseits wird durch Ausführung der Prüf-App 140 von dem elektronischen Gerät 134 der maschinenlesbare Code 120 von dem Sicherheitsdokument 110 optisch erfasst, beispielsweise durch eine Kamera oder einen optischen Scanner des elektrischen Geräts 134. Teil dieses maschinenlesbaren Codes 120 ist auch die Signatur/Siegel 124.

Die Prüf-App 140 prüft dann die Signatur/Siegel 124 auf Validität, und zwar unter Verwendung der PKI 138. Falls die Signatur/Siegel 124 verifiziert werden konnte, so prüft die Prüf-App 140 ferner, ob die Identitätsdaten 114 aus dem Ausweisdokument 102 mit den Identitätsdaten aus dem maschinenlesbaren Code 120 übereinstimmen. Wenn sowohl die Prüfung der Signatur/Siegel 124 als auch diese Prüfung auf Übereinstimmung erfolgreich waren, so wird die Information 118 geprüft, das heißt hier ob das Sicherheitsdokument 110 eine Angabe beinhaltet, wonach keine oder keine wesentliche Ansteckungsgefahr von der Person 100 ausgeht und ob ferner die Gültigkeit dieser Angabe noch nicht abgelaufen ist.

Falls sämtliche dieser Bedingungen erfüllt sind, so erzeugt die Prüf-App 140 ein Signal, welches zum Beispiel an eine Zugangskontrollvorrichtung, wie zum Beispiel ein Drehkreuz, ausgegeben wird, um einen Zugang der Person 100 in den zugangsbeschränkten Bereich zu ermöglichen.

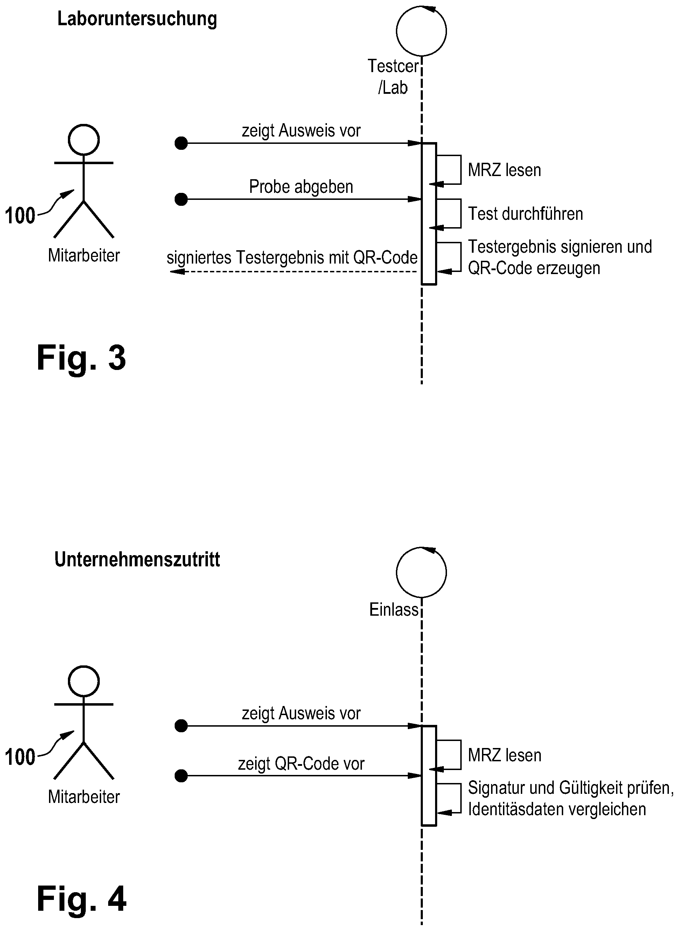

Die Figur 3 zeigt nochmals im Detail den Ablauf bei der oben genannten Laboruntersuchung. Bei der hier betrachteten Ausführungsform wird das Testergebnis, das heißt die Information 118, von dem Aussteller 112 digital signiert, was mit in den maschinenlesbaren Code 120, das heißt hier den QR-Code, eingeht.

Die Figur 4 zeigt den oben mit Bezug auf die Figur 2 erläuterten Ablauf für den Fall eines Zutritts in ein Unternehmen. Der Person 100, hier einem Mitarbeiter des Unternehmens, wird der Zutritt zum Beispiel zu einem Bürogebäude des Unternehmens, nur gewährt, nachdem dieser von der Prüf-App 140 freigegeben worden ist.

Nach Ausführungsformen der Erfindung werden die Identitätsdaten 114 zur Erzeugung des Sicherheitsdokuments 110 von dem elektronischen Gerät 116 mit der Information 118 kombiniert, indem beispielsweise die Identitätsdaten 114 an die Information 118 angehängt werden oder eine andere Reihenfolge der Konkatenierung oder Kombination dieser Information durchgeführt wird.

Nach Ausführungsformen der Erfindung werden die Identitätsdaten 114 mithilfe eines kryptografischen Verfahrens durch das elektronische Gerät 116 verarbeitet und die Kombination erfolgt dann nicht mit den Identitätsdaten 114 selbst, sondern mit dem Ergebnis dieser kryptografischen Verarbeitung. Beispielsweise wird aus den Identitätsdaten 114 ein Hashwert erzeugt und dieser Hashwert wird mit der Information 118 kombiniert und in das Sicherheitsdokument 110 eingebracht.

Zur Prüfung wird dann in analoger Art und Weise durch das elektronische Gerät 134 aus den Identitätsdaten 114, die aus dem Ausweisdokument 102 ausgelesen werden, ein Hashwert gebildet und es werden dann der so gewonnene Hashwert mit dem Hashwert aus dem maschinenlesbaren Code 120 miteinander verglichen anstelle unmittelbar der Identitätsdaten.

Nach einer weiteren Ausführungsform der Erfindung wird durch das elektronische Gerät 116 aus den Identitätsdaten 114 ein kryptografischer Schlüssel erzeugt, beispielsweise durch Bildung eines Hashwertes aus den Identitätsdaten 114. Die Information 118 wird dann mithilfe dieses kryptografischen Schlüssels verschlüsselt und dieses Chiffrat wird in den maschinenlesbaren Code 120 eingebracht.

Bei der Prüfung wird dann in analoger Art und Weise dieser kryptographische Schlüssel aus den Identitätsdaten 114 gewonnen, die aus dem Ausweisdokument 102 ausgelesen werden, um das Chiffrat aus dem maschinenlesbaren Code 120 mithilfe dieses Schlüssels zu entschlüsseln. Dies gelingt nur, wenn die Identitätsdaten 114 aus dem Ausweisdokument 102 tatsächlich mit den Identitätsdaten übereinstimmen, die Grundlage für die Erzeugung des maschinenlesbaren Codes 120 waren.

Nach einer Ausführungsform der Erfindung erfolgt alternative oder zusätzlich zu einer Verschlüsselung der Information 118 eine Verschlüsselung des Datensatzes oder des Sicherheitsdokuments 110 mit diesem kryptografischen Schlüssel, also beispielsweise mithilfe des Hashwerts der Identitätsdaten 114.

Nach einer weiteren Ausführungsform der Erfindung wird für die Erzeugung des Sicherheitsdokuments 110 ein Zufallswert verwendet, der der Person 100 von dem Aussteller 112 mitgeteilt wird. Dieser Zufallswert geht neben den Identitätsdaten 114 in die Erzeugung des kryptografischen Schlüssels ein. Dementsprechend kann eine Prüfung des Sicherheitsdokuments 110 nur erfolgen, wenn die Person 100 den Prüfer 132 bzw. dem elektronischen Gerät 134 diesen Zufallswert mitteilt bzw. eingibt. Diese Ausführungsform ist besonders vorteilhaft für den Schutz besonders sensitiver Informationen der Person 100.

### Bezugszeichenliste

- 100: Person
- 102: Ausweisdokument
- 104: Lichtbild
- 106: Personalisierungsinformation
- 108: MRZ
- 110: Sicherheitsdokument
- 112: Aussteller
- 114: Identitätsdaten
- 116: elektronisches Gerät
- 118: Information
- 120: maschinenlesbarer Code
- 122: Klartext
- 124: Signatur/Siegel
- 126: Sender
- 128: Endgerät
- 130: Drucker
- 132: Prüfer
- 134: elektronisches Gerät
- 136: Zertifikat
- 138: PKI
- 140: Prüf-App

## Patentansprüche

1. Verfahren zur Erzeugung eines Sicherheitsdokuments (110) für eine Person (100), die Inhaber eines Ausweisdokuments (102) ist, mit folgenden Schritten:
- Auslesen von Identitätsdaten der Person aus dem Ausweisdokument,
- Eingabe einer der Person zugeordneten Information (118),
- Erzeugung eines Datensatzes aus den Identitätsdaten und der der Person zugeordneten Information,
- Ausgabe des Datensatzes als Sicherheitsdokument (110), das den Datensatz als maschinenlesbaren Code (120; 124) beinhaltet.

2. Verfahren nach Anspruch 1, wobei es sich bei dem Code um einen QR-Code, Barcode, HEX-code oder Binärcode handelt und/oder wobei der Datensatz und/oder das Sicherheitsdokument durch einen Aussteller (116) des Sicherheitsdokuments signiert und/oder gesiegelt ist.

3. Verfahren nach Anspruch 1 oder 2, wobei es sich bei dem Ausweisdokument um ein maschinenlesbares Reisedokument mit einer maschinenlesbaren Zone (108) handelt, und wobei das Auslesen der Identitätsdaten durch optische Erfassung der maschinenlesbaren Zone erfolgt.

4. Verfahren nach einem der vorherigen Ansprüche, wobei zur Erzeugung des Datensatzes die Identitätsdaten und die der Person zugeordneten Information kombiniert werden, insbesondere konkateniert.

5. Verfahren nach einem der vorhergehenden Ansprüche, wobei zur Erzeugung des Datensatzes die folgenden Schritte durchgeführt werden:
- Verarbeitung der Identitätsdaten mithilfe eines kryptographischen Verfahrens welches ein kryptographisches Ergebnis ausgibt,
- Kombination, insbesondere Konkatenierung, des kryptographischen Ergebnis mit der Information,
wobei es sich bei dem kryptographischen Verfahren beispielsweise um ein irreversibles kryptographisches Verfahren, insbesondere ein HASH handelt.

6. Verfahren nach einem der vorhergehenden Ansprüche, wobei zur Erzeugung des Datensatzes die folgenden Schritte durchgeführt werden:
- Ableitung eines kryptographischen Schlüssels aus den Identitätsdaten durch ein kryptographisches Verfahren, beispielsweise durch Bildung eines HASH Wertes aus den Identitätsdaten,
- Verschlüsselung der der Person zugeordneten Information mithilfe des kryptographischen Schlüssels.

7. Verfahren nach einem der vorhergehenden Ansprüche, wobei die folgenden Schritte weiteren Schritte durchgeführt werden:
- Ableitung eines kryptographischen Schlüssels aus den Identitätsdaten durch ein kryptographisches Verfahren, beispielsweise durch Bildung eines HASH Wertes aus den Identitätsdaten,
- Verschlüsselung des Datensatzes und/oder des Sicherheitsdokuments mit dem kryptographischen Schlüssel.

8. Verfahren Anspruch 6 oder 7, mit dem weiteren Schritt der Ausgabe eines Zufallswerts für die Person, wobei in die Ableitung des kryptographischen Schlüssels neben den Identitätsdaten auch der Zufallswert eingeht.

9. Verfahren nach einem der vorherigen Ansprüche, wobei sich bei der Information um einen medizinischen Zustand der Person, einen Identifikatior eines der Person zugeordneten Gegenstandes, eine Angabe einer Befähigung oder Berechtigung der Person und/oder eine zeitliche Begrenzung handelt und/oder
wobei es sich bei der Information um einen Infektionszustand der Person bezüglich einer Viruserkrankung handelt
und/oder wobei die Information eine zeitliche Angabe hinsichtlich des Zeitpunkts der Feststellung des Infektionszustands und/oder einer Gültigkeitsdauer des Infektionszustand beinhaltet
und/oder wobei zur Feststellung des medizinischen Zustands, insbesondere eines Infektionszustands der Person, ein Analyseverfahren durch ein medizinisches Labor durchgeführt wird, und wobei es sich bei dem Aussteller des Sicherheitsdokuments um das medizinische Labor handelt.

10. Verfahren nach einem der vorhergehenden Ansprüche, wobei das Sicherheitsdokument ausgedruckt wird und/oder wobei das Sicherheitsdokument als Datei ausgegeben und an ein Endgerät der Person übertragen wird.

11. Sicherheitsdokument als Ausdruck oder als elektronisches Dokument, welches nach einem Verfahren nach einem der vorhergehenden Ansprüche erzeugt worden ist.

12. Sicherheitssystem beinhalten und ein Ausweisdokument, das einer Person zugeordnet ist, und ein Sicherheitsdokument nach Anspruch 11.

13. Elektronisches System zur Erzeugung eines Sicherheitsdokuments für eine Person, wobei das elektronische System zur Durchführung eines Verfahrens nach einem der Ansprüche 1 bis 10 konfiguriert ist.

14. Verfahren zur Prüfung eines Sicherheitsdokuments nach Anspruch 11 mit folgenden Schritten:
- Lesen des maschinenlesbaren Codes aus dem Sicherheitsdokument,
- Auslesen der Identitätsdaten der Person aus dem Ausweisdokument,
- Prüfung der Identitätsdaten aus dem maschinenlesbaren Code auf Übereinstimmung mit den aus dem Ausweisdokument ausgelesenen Identitätsdaten der Person,
- Ausgabe eines Signals zur Signalisierung der Authentizität der Information, falls die Übereinstimmung besteht und beispielsweise mit folgenden weiteren Schritten:
- Prüfung, ob die Information ein Kriterium erfüllt,
- Ausgabe eines Steuerungssignals zu Gewährung des Zugangs zu einem zugangsbeschränkten Bereich, falls das Kriterium erfüllt ist.

15. Verwendung eines Sicherheitsdokuments nach Anspruch 11 als Schutz vor der Ausbreitung einer Infektionskrankheit, insbesondere im Falle einer Epidemie oder Pandemie, wobei die Information die Angabe beinhaltet, dass die Person nicht infektiös ist, und vorzugsweise die Angabe einer Gültigkeitsdauer während derer dieser Zustand wahrscheinlich garantiert werden kann, wobei der Person Zugang zu einem zugangsbeschränkten Bereich gewährt wird, wenn die Prüfung des Sicherheitsdokuments ergibt, dass die Information authentisch und vorzugsweise noch gültig ist.
